Europäisches Patentamt

European Patent Office

Office européen des brevets

(11) Publication number: **0 352 474**
**A2**

# EUROPEAN PATENT APPLICATION

(21) Application number: **89111287.2**

(22) Date of filing: **21.06.89**

(51) Int. Cl.4: **C12N 9/92 , C12P 19/24 , C12N 11/12 , C12N 1/00 , //(C12N1/00,C12R1:07)**

The microorganism(s) has (have) been deposited with American Type Culture Collection under number(s) ATCC 53731, 53732, 53733, 53734, 53735, 53736, 53737, 53738, 53739, 53740, 53741, 53742, 53743, 53744, 53745, 53746 and 53747.

(30) Priority: **19.07.88 US 221165**

(43) Date of publication of application:
**31.01.90 Bulletin 90/05**

(84) Designated Contracting States:
**AT BE CH DE ES FR GB GR IT LI LU NL SE**

(71) Applicant: **NABISCO BRANDS, INC.**
**200 Deforest Avenue**
**East Hanover NJ 07936-1944(US)**

(72) Inventor: **Horwath, Rober O.**
**223 Bayberry Lane**
**Westprot Connecticut(US)**
Inventor: **Lloyd, Norman E.**
**188 Sawmill Road**
**Sparta New Jersey(US)**

(74) Representative: **Brauns, Hans-Adolf, Dr. rer. nat. et al**
**Hoffmann, Eitle & Partner, Patentanwälte**
**Arabellastrasse 4**
**D-8000 Munich 81(DE)**

(54) **Thermostable glucose isomerase.**

(57) This invention relates to a process for isomerizing glucose to fructose which comprises contacting a glucose-containing solution, under isomerization conditions, at a temperature between at least about 90°C to about 105°C, with a thermostable glucose isomerizing enzyme.

EP 0 352 474 A2

# THERMOSTABLE GLUCOSE ISOMERASE

The present invention relates to a process of converting glucose to fructose. More specifically, it relates to a process for the use of a novel class of glucose isomerases which are stable at high temperatures, and which are produced by thermophilic microorganisms.

Fructose is one of the sweetest sugars known, even sweeter than the more commonly used and readily available sugar sucrose. It has long been known to use the less sweet and less expensive sugar glucose as a base for conversion to the more desirable fructose.

One method commonly employed in the past has been the conversion of glucose to fructose by the use of alkaline catalysts. A number of disadvantages are associated with this type of isomerization, however. Because the alkaline catalysts are relatively unselective, undesirable by-products, such as color bodies, may be concurrently produced with the fructose; the necessary purification of the syrups to remove the unwanted products involves costly and difficult procedures.

The more preferred method of isomerization is now conversion of glucose into fructose utilizing an enzyme having glucose isomerase activity.

Extensive descriptions of the process of enzymatic conversion of glucose to fructose may be found in Hamilton, et al. "Glucose Isomerase a Case Study of Enzyme-Catalysed Process Technology", Immobilized Enzymes in Food and Microbial Processes, Olson et al., Plenum Press, New York, (1974), pp. 94-106, 112, 115-137; Antrim, et al.; "Glucose Isomerase Production of High-Fructose Syrups", APPLIED BIOCHEMISTRY AND BIOENGINEERING, Vol. 2, Academic Press (1979); Chen, et al., "Glucose Isomerase (a Review)", Process Biochem., (1980), pp. 36-41; Nordahl., A. et al. "Fructose Manufacture from Glucose by Immobilized Glucose Isomerase", Chem. Abstracts, Vol. 82, (1975), Abs. No. 110316h; and Takasaki, "Fructose Production by Glucose Isomerase", Chem. Abstracts, Vol. 81, (1974), Abs. No. 7647a.

The amount of fructose obtained from the isomerization reaction depends upon the equilibrium of the reaction. The yield of fructose can be significantly improved if the reaction is conducted at temperatures over 50° C, and preferably over 60° C. However, the higher temperatures which favor the production of higher yields of fructose may also have the unfortunate effect of denaturing the glucose isomerase, thus reducing the stability and activity of the enzyme. Therefore, for the use of a glucose isomerase to convert glucose to fructose to be exploited to its full potential, a type of enzyme with high resistance to thermal denaturation is necessary.

A number of microorganisms are known producers of glucose isomerizing enzymes. Most of these organisms are mesophiles, i.e., they have a preferred growth temperature which is generally between 18° - 45° C; the optimum temperatures for the functioning of their glucose isomerases is also usually correspondingly low. U.S. Patent No. 4,308,349 describes the use of the genus Ampullariella to produce an enzyme which can function at temperatures between 45° -85° C, although the preferred temperature for the isomerization reaction is 50° C. At least two strains of Bacillus licehniformis (ATCC 31604, U.S. Patent No. 4,348,480; and ATCC 31667, U.S. Patent No. 4,355,103) are reported to have a glucose isomerase which is optimally active at 50°, but which may retain some activity at 70° C. The glucose isomerase of the organism Flavobacterium arborescens is also known to function at temperatures as high as 90° C, although its preferred temperature range is between 60-75° C.

It would be expected that thermophilic microorganisms, i.e., those which routinely grow at temperatures of 50° or above, would produce more thermally stable glucose isomerases, and in fact a number of such useful microorganisms have been reported. Japanese Kokai 49(1974)-30588 notes the use of a series of thermophilic actinomycetes from the genera Thermoactinomyces and Pseudonocardia, all of which generally grow at temperatures of at least 50-60° C. The glucose isomerases produced by these organisms are reported to function with substantial retention of activity at 100° C. Bacillus stearothermophilus (U.S. Patent No. 3,826,714) grows normally at 55° C, and produces a glucose isomerase which is active at temperatures up to 80° C. A mutant strain of Bacillus coagulans (U.S. Patent No. 3,979,261) grows optimally at 60-65°, and its glucose isomerase may be used from 55° -85° C. without substantial loss of activity.

In connection with the present invention, there has been discovered a new type of glucose isomerase which can function at temperatures of over 90° C with thermostabilities in excess of any previously reported enzyme. Organisms yielding such enzyme have been isolated from a variety of ecosystems. Such areas include unique ecosystems in which organic substances are decomposed by an essentially aerobic, oxidative metabolic pathway, which generates substantial heat, often up to temperatures of 80° C, by the action of thermophilic microorganisms. Until this time, however, no microbe producing a glucose isomerase has been reported from such habitats.

The organisms of the present invention have been found to grow easily at 60-70° C, and their glucose isomerases, in their natural state, are functional at temperatures between at least 90-105°C.

This invention provides an enzyme capable of the isomerization of glucose to fructose characterized by possessing a half-life of twenty minutes ($T_{20}$) at a temperature of at least 93°C and preferably of about 94°C and higher. This invention also provides the enzyme capable of the isomerization of glucose to fructose, isolatable from a member of the genus Bacillus, characterized in possessing a half-life of 20 minutes ($T_{20}$) at a temperature of at least 93°C and immobilized onto an insoluble carrier.

In a further embodiment the invention provides a process for isomerizing glucose to fructose which comprises contacting a glucose-containing feed liquor containing from 20 to about 65 weight percent glucose with a glucose isomerase at a temperature of from about 90°C to about 130°C at a pH from about 3.5 to about 8 and a contact time of from about 10 seconds to about 5 hours to convert at least about 53 to about 60 weight percent of the glucose present in said liquor to fructose with no substantial formation of psicose, which comprises employing a glucose isomerase possessing a half-life of twenty minutes ($T_{20}$) at a temperature of at least 93°C.

In a final embodiment the invention provides an enzyme capable of the isomerization of glucose to fructose characterized by possessing a half-life of twenty minutes ($T_{20}$) at a temperature of at least 93°C.

In the accompanying drawings,

Figure 1 illustrates an Arrhenius plot displaying the effects of temperature on the stability of the immobilized glucose isomerase of U.S. 3,788,945.

Figure 2 illustrates a plot of $L_T$ vs temperature based on the data in Figure 1.

Figure 3 illustrates a plot of $(l_T/l_{80})(K_{f80}/K_{fT})$ vs temperature for the data of Figure 2 and gives information on temperature vs half-life.

Figure 4 shows maps of the various localities in which the microorganisms of the invention have been found.

The present invention provides a thermostable glucose isomerase which is useful in the high temperature conversion of glucose to fructose.

As mentioned above the levels of fructose achievable by the isomerization of glucose with glucose isomerase is limited by the equilibrium of the isomerase reaction. At 65°C., the equilibrium of the reaction stands at approximately 51% fructose by weight from a starting substrate of pure dextrose. When refined glucose liquor is used as the substrate (containing up to about 6% nonmonosaccharides by weight) and allowing for a reasonable residence time in the enzyme reactor, an approximately 50% fructose syrup is the highest fructose content which can be obtained (on a dry basis) by the prior procedures referred to. To attain syrups of higher fructose content fractiona tion systems must be employed which add greatly to the cost of the final product. At higher temperatures, however, the equilibrium becomes more favorable. For example, an enzymatic glucose isomerase process capable of being operated at temperatures of from about 90°-130°C. could be used to directly provide high fructose corn syrups (HFCS) containing 53-60 weight percent fructose on a dry basis thereby eliminating the need for fractionation and recycle. However, the tendency of known glucose isomerase systems to undergo thermal denaturation with an accompanying sharp reduction in activity has thus far frustrated attempts to utilize higher temperature regimes to force equilibrium of the isomerization further in favor of fructose.

U.S. Patents 4,410,627, 4,411,996 and 4,567,142 teach inter alia that careful control of reaction conditions as well as the chemical stabilization of the enzyme can be employed to provide a product of containing up to about 65 weight percent fructose.

The maximum degree of conversion of glucose to fructose that can be attained is governed by the thermodynamic equilibrium between glucose and fructose which in turn is dependent upon the temperature at which the isomerization is conducted. Very careful analysis of equilibrium mixtures of glucose and fructose has established the following relationships.

$$F_e = 100K/(K + 1) \qquad (1)$$

$$1nK = -755/(T + 273) + 2.3005 \qquad (2)$$

where $F_e$ is the % fructose at equilibrium based on total weight of glucose and fructose, T is the temperature (°C) at which isomerization is conducted, and K = fructose over glucose equilibrium constant.

Actual contact time between the glucose-containing syrup and isomerase in a reactor can generally be reckoned by reference to the following formula when a reactor containing an immobilized form of isomerase is used.

$$t_a = (CV/kA)ln((F_e - F_o)/(F_e - F)) \qquad (3)$$

where:

$t_a$ = the actual contact time

C = concentration of glucose and fructose

V = The free volume of fluid in the reactor (volume of reactor minus the volume occupied by the

immobilized enzyme particles)

$F_e$ = fraction of fructose in the glucose plus fructose mixture at equilibrium when at the isomerization temperature

$F_o$ = fraction of fructose (based on G + F) at the entrance to the reactor

F = fraction of fructose (based on G + F) in the solution exiting the reactor

k = reaction rate constant for isomerization at the isomerization conditions

A = activity of isomerase in the reactor

Values for k for immobilized isomerase range from about 0.07 to about 5 g hr$^{-1}$ at temperatures from 90° C to 140° C respectively. This relationship shows the need to minimize contact time at high temperature by using packed beds of high activity per unit volume.

In commercial practice, however, fructose-containing syrups are not manufactured from pure glucose. Rather, starch hydrolysates (as prepared in the above mentioned references) are used as the glucose source and these invariably contain non-glucose and non-fructose saccharides (hereinafter referred to as oligosaccharides of D.P. of up to about 5) derived from incomplete hydrolysis of starch, and the reversion of glucose. Typically these constitute from 3% to 8% of the total dry weight of the saccharides derived by starch hydrolysis. It is therefore necessary, when reckoning the temperature at which isomerization is to be conducted, to allow for any oligosaccharide contained in the glucose liquor as well as other factors such as the total dry basis fructose content to be attained, formation of psicose and other non- glucose and nonfructose products during the effective contact time of the glucose liquor and the isomerase. Relationships for the calculation of isomerization temperature are shown below:

$$T = 755/(2.3005 - \ln K) - 273 \quad (4)$$

$$K = F_e/(100 - F_e) \quad (5)$$

$$F_e = 10,000(M + C)/(100Q - PQ) \quad (6)$$

where:

T = isomerization temperature (° C)

$F_e$ = equilibrium fructose content (% based on total glucose + fructose) at temperature T.

M = % fructose dry basis required in the isomerized product.

C = % psicose + other degradation products formed during the effective isomerization contact time.

Q = % of equilibrium attained during isomerization reaction.

P = % oligosaccharide content of glucose liquor.

Typically, less than 1% and preferably less than 0.5% psicose and other degradation products will be formed and 99.5% of equilibrium can be attained. Therefore, to prepare syrups with 55.5% fructose (dry basis), the following isomerization temperatures are required for glucose liquors of the indicated oligosaccharide contents.

| Oligosaccharide in Glucose liquor (% dry basis) | Isomerization Temperature (° C) |
| --- | --- |
| 0 | 95.7 |
| 1 | 99.1 |
| 2 | 104.3 |
| 3 | 108.9 |
| 4 | 113.8 |
| 6 | 124.3 |
| 8 | 136.1 |

The accepted article of commerce contains on the average, 55.5% fructose on a dry basis. This is so because at this fructose level, high fructose corn syrup (HFCS) attains equal sweetness with sucrose on a weight for weight dry basis. Moreover, HFCS of 55% fructose content is firmly established as the article of commerce that is used interchangeably as a total or partial replacement for sucrose in many food products and especially in carbonated soft drinks. Owing to the complexities inherent in delivering, storing, metering and formulating HFCS into food products, there is a universal demand for uniformity of product from one HFCS manufacturer to another so that product from different supply sources can be used interchangeably and simultaneously. Therefore, fructose level of 55-56% dry basis has attained special significance as a target level in the technology associated with HFCS manufacture.

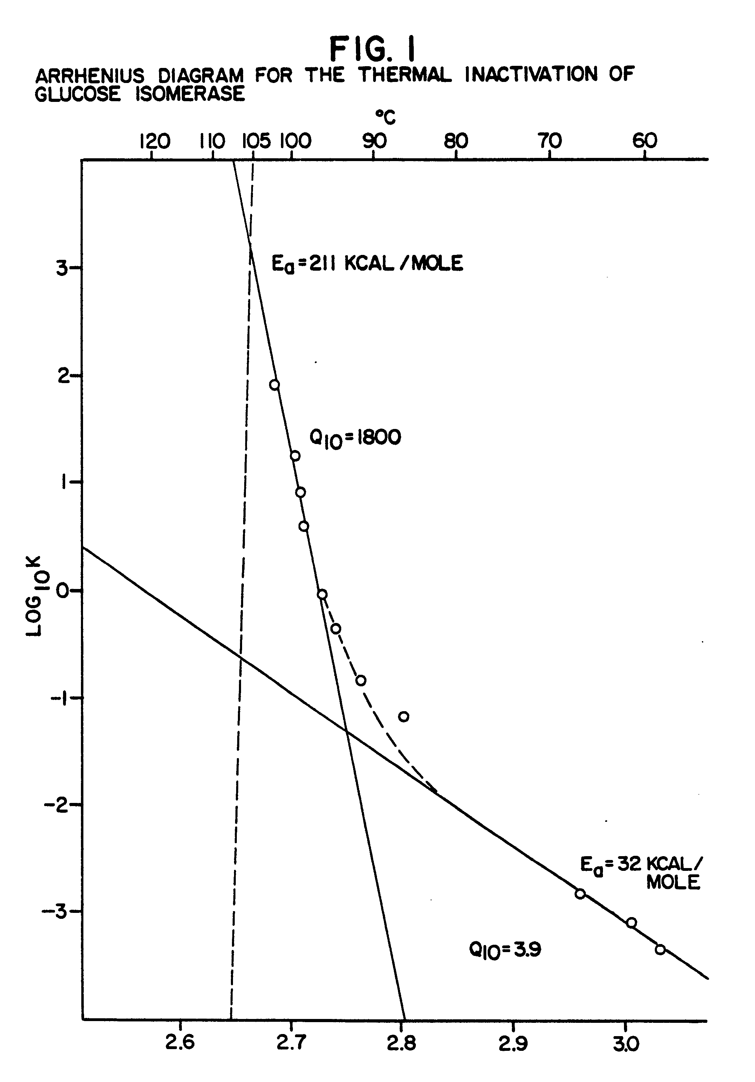

As indicated by the foregoing discussion, although a variety of parameters may be the targets for

4

improving the isomerization process, the enzyme itself remains a primary focus for process improvements, particularly with regard to thermal stability. Figure 1 shows an Arrhenius plot displaying the effects of temperature on the stability of glucose isomerase (Streptomyces rubiginosus immobilized on DEAE-cellulose according to the teachings of U.S. Patent 3,788,945) under conditions normally used for isomerization, i.e., at optimum pH (6.5 - 7.5), 50% glucose + fructose concentration, 2mM Mg + + and 2-3 mM $NaHSO_3$. These stability data are typical of prior art glucose isomerases in immobilized form. The plot shows half-life vs. temperature and reveals that two different inactivation processes are important in different temperature ranges. Below about 80°C, inactivation is due almost entirely to chemical degradation reactions with a temperature dependence characteristic of chemical processes showing a four-fold acceleration rate for every 10°C rise in temperature (i.e., $Q_{10}$ = 4). The range between about 80°C and 95°C shows a transition to a region where a different inactivation process is predominant such that above about 95°C, inactivation rate is enormously accelerated by temperature and $Q_{10}$ reaches the phenomenally high value of 1800. The activation energy for thermal denaturation (E*) generally ranges from about 90 to 300 k cal/mole. In this region, a mere 1°C. increase in temperature more than doubles inactivation rate. Such behavior with regard to temperature is commonly observed with enzymes and is regarded as due to the tendency of enzymes (which are proteins) to undergo denaturation, a physical process wherein the active conformation is lost due to thermal effects.

Since the efficiency with which glucose isomerase can be used to convert glucose to fructose is directly proportional to isomerase half-life (inversely proportional to inactivation rate) it is appreciated that temperature is an all-important consideration in the usefulness of glucose isomerase for this purpose. For this reason, the present art tends to emphasize use of isomerase at the lowest possible temperature consistent with avoiding microbial contamination in the immobilized isomerase reactors commonly employed. Thus, isomerization is currently conducted at 55-60°C where isomerase half-lives of 1000-2000 hours are observed. In contrast, half-life of the prior art isomerase depicted in Figure 1 is a mere 29 minutes at 95°C and at 100°C is only one minute. It is therefore evident that temperatures at which thermal denaturation becomes a dominant factor must be avoided in isomerization processes, and further, that even small increases in temperature in the thermal denaturation range are extremely critical with regard to half-life and enzyme efficiency.

As mentioned above, U.S. Patents 4,410,627 4,411,996, and 4,567,142 set forth the minimum criteria for the high temperature isomerization of glucose to achieve 55 percent fructose by a direct isomerization process. The significance of achieving syrup compositions with 55% fructose dry basis is also discussed therein. Because of the influence of temperature on the glucose/fructose thermodynamic equilibrium, a minimum temperature of 87°C must be employed to reach 55% conversion starting with pure glucose and carrying the reaction to the equilibrium point. Because equilibrium is only approached in theory and pure glucose is impractical as a starting material for such reactions, minimum temperature for achieving 55% fructose by direct isomerization is about 90°C and preferably about 100°C as disclosed in the above cited patents. In determining the utility of glucose isomerase for 55% or more fructose, it should be appreciated that various immobilization techniques and/or chemical modifications are capable of increasing the ability of isomerase to resist thermal denaturation such that the critical temperature range at which denaturation becomes the dominant mechanism of enzyme inactivation is shifted to a higher temperature by several degrees. As a measure of an enzyme's tendency to resist thermal denaturation, the "$T_{20}$" convention has been adopted. $T_{20}$ is the temperature expressed in °C at which a 20 minute half-life is observed. A procedure for the determination of the $T_{20}$ of glucose isomerase is set forth below. The $T_{20}$ of S. rubiginosus C-3 isomerase as disclosed below is 89.7°C. After immobilization on DEAE-cellulose, the $T_{20}$ is increased to 96°C. Other stratagems for increasing the resistance of enzymes to thermal denaturation are discussed in U.S. Patents 4,411,996 and 4,567,142. It is important to realize that such methods for stabilizing enzymes are limited with respect to the degree of stabilization that can be imparted in terms of the effect on $T_{20}$. Therefore, glucose isomerases that are inherently more thermostable to begin with (by virtue of their primary, secondary and tertiary protein structure) even by a few °C (in terms of $T_{20}$) have enormous advantage over enzymes of lower inherent stability for purposes of constructing high temperature isomerization reaction systems for operation at 90°C and higher, since, as discussed above, an increase of only 1°C in $T_{20}$ can result in a doubling of half-life. Table I shows the thermostabilities of the prior art glucose isomerases. The least thermostable of these displays a $T_{20}$ of only 84.2°C. The most widely used glucose isomerases (from Streptomyces sp., Bacillus coagulans, Arthrobacter sp. & Actinoplanes missouriensis) show $T_{20}$ in a narrow range of values (89.3-91.7°C). The most thermostable isomerase from the prior art is from Ampullariella sp. with a $T_{20}$ of 92.8°C.

It has now been discovered that some glucose isomerases from thermophilic Bacillus sp. have thermostabilities much greater than any of the prior art isomerases thus far disclosed. This is very surprising in view of the large number of glucose isomerases so far revealed from a great variety of microbial sources (for a discussion see Chen, W-P, Proc. Biochem., Jan/July, 1980, pg. 30-41). It is estimated, for example, that no less than 60 different glucose isomerases have been disclosed thus far. Among the isomerases derived from Bacillus sources are the three prior art enzymes shown in Table II. Two of these have been described as being extremely thermostable (see U.S. Patent 3,826,714 and U.K. Patent 1,457,177). As shown herein, these enzymes display only ordinary thermostability as measured by the definitive $T_{20}$ criterion, and in fact, are far less thermostable than the isomerase of the instant invention.

## TABLE I

## Thermostability of Prior Art Isomerase

| | $T_{20}$ (°C) |
|---|---|
| Streptomyces acidodurans (A.E. Staley)[a] | 84.2 |
| Pseudonocardia thermophila (Takasaki)[b] | 89.3 |
| Streptomyces rubiginosus 4-A (NBI) | 89.3 |
| Streptomyces rubiginosus C-3 (NBI) | 90.2 |
| Actinoplanes missouriensis (Anheuser Busch)[c] | 90.7 |
| Arthrobacter 18C (NBI)[d] | 91.7 |
| Streptomyces rubiginosus C-3 + $Co^{++}$ (NBI) | 92.7 |
| Ampullariella sp. (Dow Corning)[e] | 92.8 |

| | Accession number | Citation |
|---|---|---|
| a. | NRRL 11489, 11492, 11495, 11496 | 4,459,354 (U.S. Patent) |
| b. | IFO No. 12133 | SHO 49 (1974) - 30588 (JAPAN) |
| c. | NRRL B-3342 | 3,992,262 (U.S. Patent) |
| d. | ATCC 21748 | 4,593,001 (U.S. Patent) |
| e. | ATCC 31351-31354 | 4,308,349 (U.S. Patent) |

## TABLE II

### Thermostability of Prior Isomerase from Bacillus sp

|  | $T_{20}$ (°C) |
|---|---|
| Bacillus stearothermophilus (SnamProgetti)[a] | 88.6 |
| Bacillus stearothermophilus (CPC)[b] | 89.4 |
| Bacillus coagulans (Novo)[c] | 90.1 |

| | Accession number | Citation |
|---|---|---|
| a. | CBS 40473 | 1,457,177 (U.K. Patent) |
| b. | ATCC 21365 | 3,826,714 (U.S. Patent) |
| c. | NRRL 5649-5666 | 3,979,261 (U.S. Patent) |

As discussed in the foregoing, the effect of temperature on the inactivation rate of enzymes is exceedingly high in the temperature range where thermal denaturation effects are predominant. Activation energies for denaturation of 100-300 kilocalories/mole are the rule for the glucose isomerases investigated herein. The effect of temperature on the rate of isomerization on the other hand follows the usual pattern seen for chemical reactions and is most typically 13 kilocalories/mole for glucose isomerase catalyzed reactions. The extreme sensitivity of denaturation rate to temperature (as opposed to the relative insensitivity of catalytic rate) is used as the basis for ranking thermostability on a temperature scale.

The system is based on the following kinetics which are generally valid for glucose isomerase.

$$dI/dT = kE(I_e - I)/C \qquad (7)$$

$$dE/DT = -0.693E/tau \qquad (8)$$

where:

$I$ = degree of isomerization (mole fraction of fructose based on glucose + fructose)

$I_e$ = I at equilibrium

$k$ = reaction rate constant for isomerization

$E$ = concentration of isomerase

$C$ = total concentration of glucose + fructose

$tau$ = isomerase half-life

$\frac{dI}{dt}$ = isomerization rate

$\frac{dE}{dt}$ = isomerase inactivation rate

Combining (7) & (8) and integrating leads to the following expression. $L_T = (k_T E_0 t/C) ((1 - 0.5^n)/(0.693n))$ (9)

where $L_T = 1n(I_e/(I_e - I_T))$ (10)

and

$L_T$ = activity function at temperature T as defined by (10)

$E_0$ = enzyme concentration at the start of heating period

$t$ = duration of heating period

$n$ = t/tau

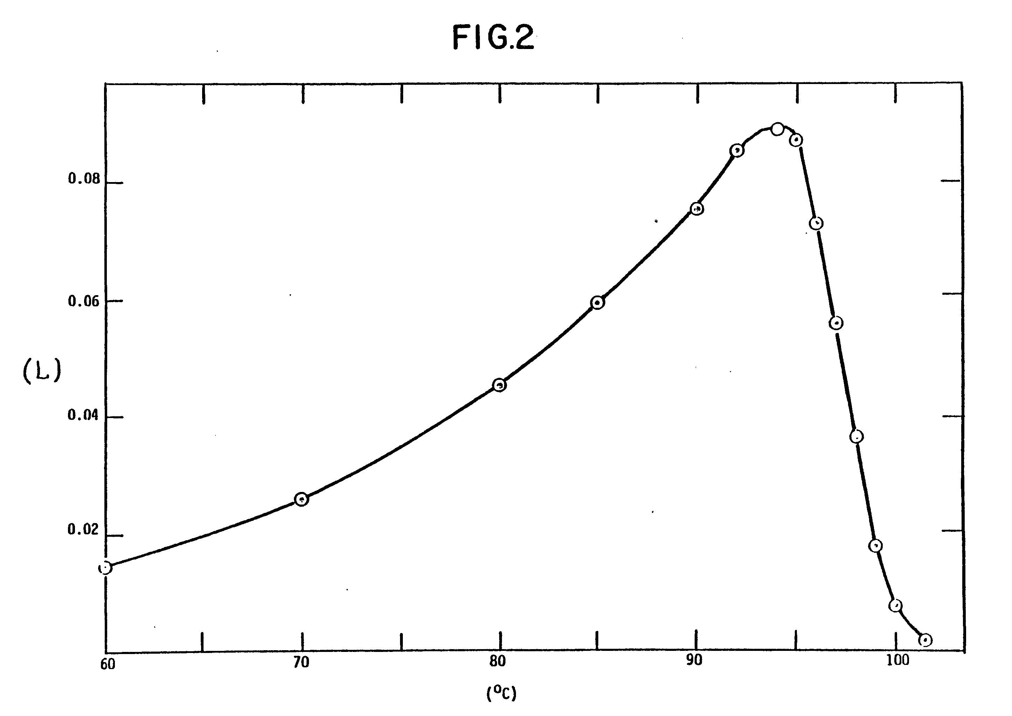

Figure 2 shows a plot of $L_T$ vs. temperature based on the data shown in Fig. 1 for a 40 minute heating period. Isomerization increases as temperature is increased until the critical temperature range is reached after which isomerase denaturation becomes predominant and isomerization rate diminishes to near zero over a mere 5°C range. When the temperature is below the critical range at which denaturation predominates (for example, below about 90°C in Fig. 2) the term within the brackets in Eq. 9 is equal to 1 (tau t). Thus, a reference temperature is chosen below the critical range such that $L_R = k_R E_0 t/C$ (11)

Dividing (9) by (11) gives

$$A_r = L_T k_R/(L_R k_T) = ((1 - 0.5^n)/(0.693n)) \qquad (12)$$

where:

$A_r$ = relative activity

The above shows that $A_r$ is a function only of the duration of the heating period, t, and of enzyme half-life.

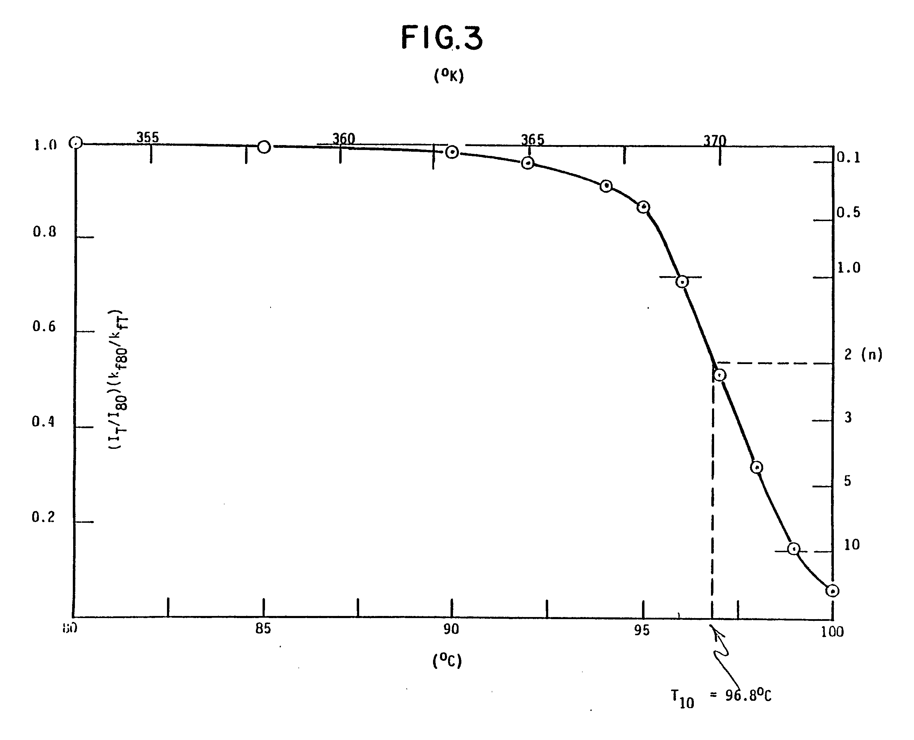

Thus, a plot of $A_r$ vs. temperature over the critical temperature range (for the example in Fig. 2, from about 95 to about 100°C) gives information on temperature vs. half-life. An example is shown in Fig. 3 for the data in Fig. 2 assuming that t = 40 min., $E_o$ = 0.5 IGIU/ml, $i_e$ = 0.565, and C = 0.2 g/ml.

The temperature at which n = 2 (tau = 20 min.) is determined by interpolation of the plot and the value taken as an estimate of $T_{20}$.

Specifically, the procedure for determining $T_{20}$ for isomerases discussed herein was as follows:

1. Buffer solution and glucose solution are prepared in distilled water as follows:

<u>Buffer solution</u>: sodium maleate buffer 25 mM; $MgSO_4$, 10mM; adjust to pH 6.5.

<u>Glucose substrate solution</u>: glucose (reagent) 400g/L; sodium maleate buffer 25 mM; $MgSO_4$ 10mM; stabilizer (e.g., $Fe^{++}$, $Mn^{++}$, or $Co^{++}$) concentrations as specified; adjust the pH to 6.5.

2. Two grams harvested wet cell cake are suspended in a 2 ml buffer solution and sonicated for a total of 75 seconds in 15-second bursts using a Branson sonifier (W-350). The sonicated cells are centrifuged and filtered through 1.2 micron Gelman filter (Acrodisc Cat. No. 4190).

3. The glucose isomerase activity in the cell extract is assayed by the method described by Lloyd, et al. [Cereal Chem., 49 544-554 (1972)] and adjusted to a concentration of 1.0-1.5 IGIU/ml.

4. 100 ul thin-walled capillary glass micropipets (VWR Cat. #53432-921) are washed with chromic acid solution, rinsed with deionized water, dried and bottom ends flame-sealed.

5. The adjusted glucose isomerase solution is chilled to approximately 1°C and is mixed with an equal volume of glucose solution (also at 1°C) and distributed into the 100 ul capillary micropipets which are then flame-sealed at top end, (while still at 1°C) and incubated in heating baths at selected temperatures for 40 minutes. The heating baths should be capable of maintaining temperature to within ±0.1°C. Temperature of the heating baths is set at 1°C intervals over the critical temperature range of the enzyme (for the example in Fig. 3, this would be between 93° and 100°C). One bath is set at the reference temperature which is optionally set at about 10°C below the $T_{20}$.

6. Blanks are prepared whereby buffer solution is mixed in 1:1 ratio with glucose solution, sealed into capillary micropipets and incubated 40 minutes in the same heating baths as the enzyme samples.

7. The reaction is terminated by immersing the micropipets in a salt-ice bath at 0°. The micropipets are then opened, and 65 ul from each micropipet is added to 2 ul of 1 N HCL to stabilize the isomerase contents until the glucose and fructose concentrations are assayed by HPLC. Degree of isomerization ($I_T$) is calculated at each temperature

$$I_T = F/(F + G) \qquad (13)$$

F = % fructose in sample - % fructose in blank

G = % glucose in sample

Activity function at each temperature is calculated ($L_T$) $L_T = \ln(I_e/(I_e - I_T))$ (14)

where: $I_e = K/(K + 1)$ (15)

$\ln K = -755/(T + 273) + 2.3005$ (16)

T = temperature in °C

Relative activity ($A_r$) at each of the test temperatures may be calculated by:

$$A_r = (L_T/L_R)(k_R/k_T) \qquad (17)$$

where:

$L_T$ = activity function at temperature, T.

$L_R$ = activity function at reference temperature, R.

$k_r$ = isomerization rate constant at reference temperature, R.

$k_T$ = isomerization rate constant at test temperature, T.

Isomerization rate constants are calculated using the following expression:

$$\ln k = -6654/(T + 273) + 15.957 \qquad (18)$$

Relative activity is plotted vs. temperature as shown in Fig. 3 and $T_{20}$ determined by interpolation from the coordinates at which $A_r$ = 0.5411, (n = 2).

## Strains Useful for Practicing the Invention

Strains of bacteria which produce glucose isomerase having the novel characteristics described herein, while not ubiquitous, are certainly cosmopolitan. The organisms are recoverable from a variety of ecosystems in Texas, Montana, California, Pennsylvania, as well as in Puerto Rico. The existence of certain soil conditions will, to a large extent, predict the presence of these microorganisms. Among the suitable soil characteristics for growth of these bacteria are high humidity, a neutral pH, between about 5.5-8, and high surface temperature; also important is a low enzyme inactivating mineral content and low calcium content. Table III presents a list of the types of habitat in which the microorganisms have been found; the skilled artisan will also readily recognize other potential sources of the bacteria in view of the habitats demonstrated here.

TABLE III

Localities at Which Thermophilic Bacillus Have Been Recovered

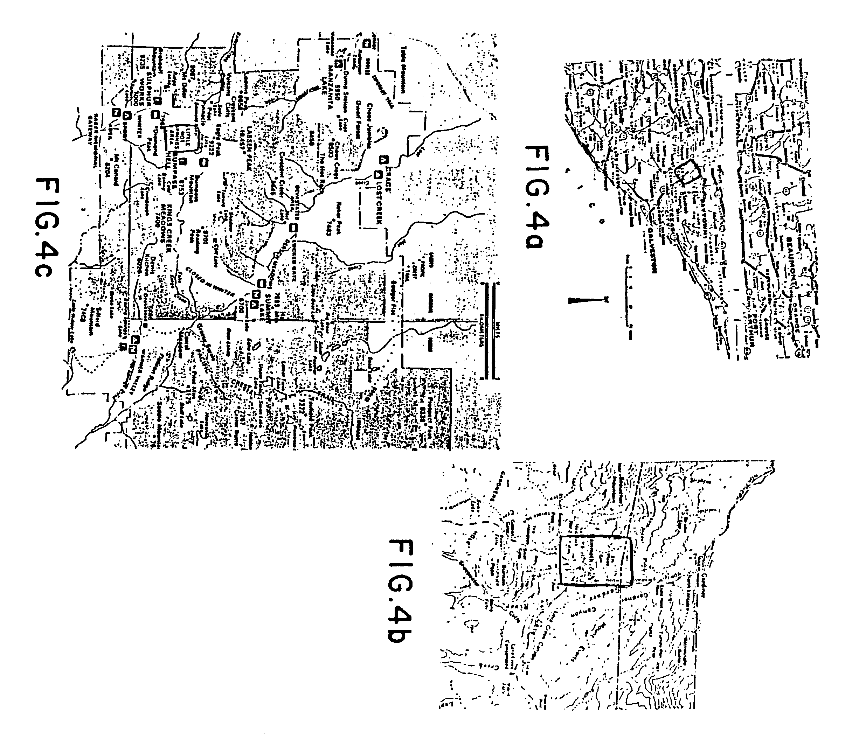

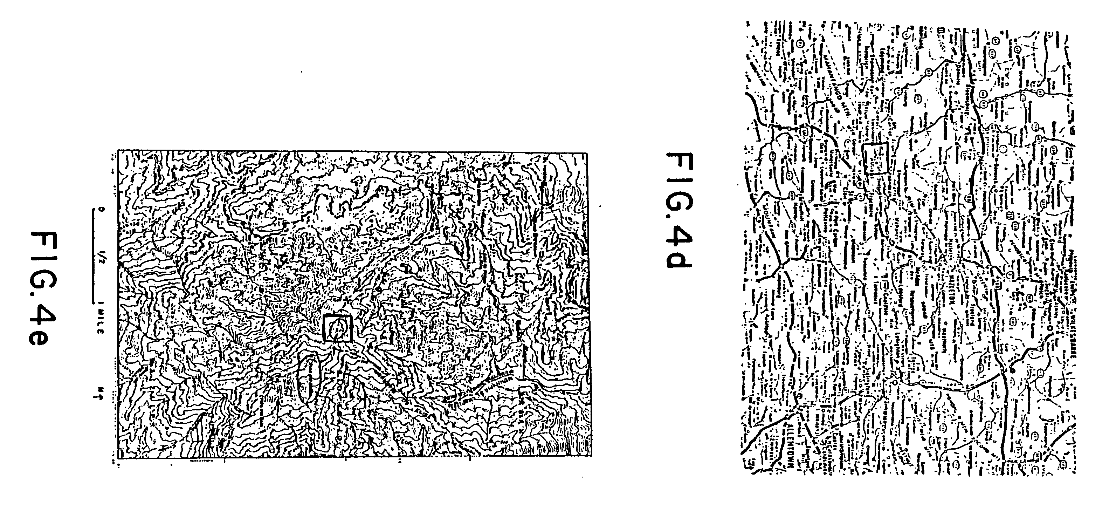

Texas: Along Route 45, approximately 20 miles from Houston, heading toward Galveston, in a plowed rice field with very black soil; the soil temperature at midday on a sunny day reached well over 100° F (Figure 4, Map A); June; 1982, and January, 1984.

Wyoming/Montana: In Yellowstone National Park, in the Mammoth area; within 1-2 miles from the main road, in the soil bordering the many hot springs in that area (Figure 4, Map B); July, 1983.

California: In Lassen Volcanic National Park, in the soil bordering the hot springs in the valley of the Little Hot Springs Area, within Bumpass Hell (Figure 4, Map C); September, 1984.

Pennsylvania: Between the towns of Centralia and Ashland, just off the main road, within a two mile span, in the soil bordering steam vents which are visible from the road (Figure 4, Map D); November, 1984.

Puerto Rico: In Luquillo Experimental Forest, a rain forest of El Yunque, along the main trail leading to Pico del Oeste, approximately $\frac{1}{2}$ mile northwest of the peak, in rotting vegetation (Figure 4, Map E); May, 1984.

Once collected, soil samples are screened for the presence of thermostable glucose isomerase-producing microorganisms according to the following protocol:

| Medium used to propagate the isolates in primary, secondary and tertiary screening : | |
| --- | --- |
| Xylose | 0.5% (autoclaved separately) |
| Corn Steep Liquor (48% Solids) | 1% |
| Peptone | 0.5% |
| NaCl | 0.2% |
| yeast extract | 0.1% |
| $MgSO_4 7H_2O$ | 0.024% |
| $CoCl_2$ | 0.0024% |
| Agar | 2.5% |
| $K_2HPO_4$ | 1.1% |
| $KH_2PO_4$ | 0.6% |
| pH | 6.8% |

## Primary Screening:

Soil Sample

↓

Large xylose agar plates were inoculated with soil suspension and incubated at 55°C for 16 hours.

↓

Singe colonies were picked, streaked on small petri-dishes (100 X 15mm) and incubated at 55°C for 16 hours. Single colonies developing from these streaks were found to be pure.

↓

The pure isolates were inoculated onto xylose agar slants for secondary screening and maintenance.

Secondary Screening:

Isolates on slants were streaked on xylose agar petri-dishes and incubated at 55°C for 16 hours.

The growth from each isolate was scraped into test tubes containing 1.5ml of maleate buffer (pH 6.5) and sonicated for 10 seconds. Then 1.5ml 40% glucose substrate solution described previously was added, mixed and the test tubes were incubated for 1 hour at 98°C.

After the incubation the isomerization reaction in the test tubes was stopped by adding 100 ul 1N HCl and the samples were assayed for fructose.

The assay for fructose was done using a Technicon Autoanalyzer. Fructose reacts with cysteine-carbazole sulfuric acid reagent and the colorimetric response is measured using a colorimeter and recorder. (Lloyd, N.E., et al., Cereal Chem., 49, 544-554 [1972]).

Isolates showing higher than average fructose amounts go on to tertiary screening.

Tertiary Screening:

The best isolates from secondary screening results were streaked on xylose agar petri-dishes and incubated at 55°C for 16 hours.

The growth was scraped into 2ml maleate buffer (pH 6.5), sonicated for 45 seconds (15-second bursts), centrifuged and filtered using 1.2 micron filter membrane.

The cell extracts thus obtained were assayed for glucose isomerase activity by the Technicon method.

The isolated glucose isomerases were evaluated by the capillary micropipet method previously described. After incubation at 98°C for 40 minutes, the reaction was stopped by acid, and the glucose-fructose content assayed by HPLC.

Samples are automatically injected into the HPLC, and the sugars are separated by a carbohydrate column (#84038 Waters Assoc. Inc.) using acetonitrile/water (80/20) as a mobile phase at 30°C.

Waters R-401 differential refractonater is used as detector, and M-730 Data Module as printer/plotter. The results are compared to the blank (no enzyme) and isolates showing activity are further evaluated. More than 14,000 isolates have been investigated and when selected cultures were evaluated by the $T_{20}$ test described above, a number have shown $T_{20}$ values exceeding any of the prior art isomerases (See Table V).

Enzymes possessing $T_{20}$ values exceeding 93°C are useful in practicing the subject invention. Particularly useful are enzymes in the range from at least 93°C, or about 94°C, to about 125°C. Also particularly useful are enzymes in the range from about 94°C to about 115°C.

The following is a summary of the relevant taxonomic characteristics of a number of the thermophilic isolates of the present invention which indicates that they are of the genus Bacillus.

## TABLE IV

| Isolate Characteristic | 2197* | 3309* | 6536* | 6626* | 6653* | CPC Patent (ATCC 21365) | Snamprogetti (CBS 40473) | B. stearothermophilus (ATCC 12900) |
|---|---|---|---|---|---|---|---|---|
| Voges Proskauer | + | + | - | - | - | - | - | - |
| Anaerobic growth | | | - | - | - | - | | - |
| Motility (37°C) | - | + | + | + | + | + | poor | + |
| Starch hydrolysis | | | + | + | + | - | + | + |
| Growth in 7% NaCl | | | - | - | - | - | | + |
| lysozyme | | | - | - | - | - | | - |
| Na azide | | | - | - | - | - | | - |
| SAB dextrose (pH 5.7) | | | - | - | - | - | - | - |
| Indole | | | - | - | - | | | - |
| Gram reaction | | + | +/- | +/- | +/- | + | - | - |
| Catalase | | + | + | + | + | + | + | + |
| Citrate | | + | - | + | - | + | | - |
| Nitrate reduction | | | - | + | + | + | | + |
| Spores | | | T/ST | T/ST | T/ST | T/ST | T/ST | T/ST |
| $H_2S$ | - | | - | - | - | - | | - |
| Maximum Growth Temp., °C | 60 | 60 | 67 | 65 | 70 | | 70 | 70 |
| Minimum Growth Temp., °C | 24 | 24 | 40 | 38 | 36 | | 36 | 40 |
| Gelatin liquified | | | + | + | + | + | + | - |
| Glucose acidified | | | + | + | + | + | + | + |

T = Terminal
ST = Sub Terminal
* = Isolate of Table IV

TABLE V

| T$_{20}$ Values of Enzymes Useful in Practicing The Subject Invention | | |
|---|---|---|
| Isolate No. | Soil Source * | T$_{20}$(°C) |
| 12,117 | Pennsylvania | 93.9 |
| 12,143 | Pennsylvania | 95.1 |
| 12,137 | Pennsylvania | 95.5 |
| 3289 | Wyoming/Montana | 96.8 |
| 3309 | Wyoming/Montana | 97.3 |
| 12,126 | Pennsylvania | 97.3 |
| 10.050 | California | 97.3 |
| 4910 | Wyoming/Montana | 97.4 |
| 12,122 | Pennsylvania | 98.1 |
| 2197 | Texas | 98.1 |
| 12,124 | Pennsylvania | 98.2 |
| 7652 | Puerto Rico | 98.3 |
| 10,054 | California | 98.3 |
| 12,119 | Pennsylvania | 98.7 |
| 6536 | Puerto Rico | 98.8 |
| 6626 | Puerto Rico | 99.2 |
| 6653 | Puerto Rico | 100.0 |

* From Table III above

The organisms of the present invention may be grown in a xylose/corn steep liquor medium. Xylose must be present in the medium for the production of glucose isomerase. Also, the addition of magnesium to the medium is preferred to promote growth of the organism. The organisms are capable of growth at temperatures between about 24°C and 70°C, but optimal growth occurs between 50°C and 62°C. Under these conditions, yields of glucose isomerase range from about 0.5 to 1.6 IGIU/ml. The high temperatures at which the organism grows provide an added advantage in that they generally allow for a relatively short period of incubation. Usually 6-10 hours is a sufficient period of time for production of satisfactory levels of enzyme.

The growth medium is prepared as follows:

| | |
|---|---|
| xylose | 1% (filter sterilized) |
| yeast extract | 0.1% |
| corn steep liquor (CSL) (48% solid) | 2% |
| NaCl | 0.2 g |
| casamino acid | 1% |
| MgSO$_4$7H$_2$O | 0.03% |
| K$_2$HPO$_4$ | 1.1% |
| KH$_2$PO$_4$ | 0.6% |
| Pluronic L61 surfactant (BASF Wyandotte Corp.) | 1 drop |
| pH | 6.0 |

The organisms are grown in shake flasks at 200 RPM at a temperature of 55°C, followed by propagation in laboratory-scale fermentors with 1:1 v/v aeration, a temperature of 55°C, and agitation at 600 RPM.

The enzyme so produced is operable at a wide range of temperatures. Its principle advantage, however, is that its optimum activity occurs at temperatures much higher than those associated with previously known organisms. Furthermore, the enzymes may be used in an immobilized state, for example, as in the processes described in U.S. Patent Nos. 4,411,996 or 4,410,627. Conditions under which isomerization may be carried out vary, but best results are obtained when the glucose containing solution is in the range of 5 to 60% by weight, and preferably in the range of from about 30 to 50%. The pH of the glucose containing solution should be maintained within the range of 6.0 to 7.0. In the glucose containing solution may also be included various ion activators and/or stabilizers such as magnesium, iron or cobalt, or combinations thereof.

The scope of the present invention is illustrated by the following Example, which is not intended to be limiting upon the scope of the invention.

## EXAMPLE 1

This example illustrates the purification and immobilization of a thermostable glucose isomerase useful in practicing the invention.

### Partial Purification of Isolate 6653

Extraction – Frozen cells were suspended at a concentration of 1 g cells/4 ml in a solution containing 50 mM maleate, pH 6.5, 20 mg lysozyme/4 ml, 200 PPM quaternary amine such as Benzalkonium Chloride (BTC). The suspension was stirred vigorously for 4 hours at 45°C and the cellular debris removed by centrifugation.

Heat Treatment - The extract was heated to 80°C and held 10 minutes after which a heavy white precipate was removed by centrifugation.

Ultrafiltration - Clarified extract was ultrafiltered and diafiltered with a 100,000 Molecular Weight Cut-Off (MWCO) membrane. Enzyme was retained.

DEAE-Cellulose chromatography - Ultrafiltration concentrate was applied to a DEAE-cellulose column equilibrated at pH 7 with 25 mM Tris buffer. A NaCl gradient was applied eluting the enzyme off at about 0.26 M NaCl. Active fractions were pooled and desalted on a 50,000 MWCO UF membrane.

Gel Filtration Chromatography - A portion of the concentrated DEAE-cellulose eluate was applied to a TSK HW-35 (Merck, hydrophilic vinyl resin) column and eluted with 25 mM phosphate buffer.

An approximate estimate of purification across each purification step is as follows:

|  | Specific Activity |
| --- | --- |
| Cells | 2-5 IGIU/g wet cell paste |
| Extraction | 0.17 IGIU/mg protein |
| Heat Treatment | 0.6-0.8 IGIU/mg protein |
| Ultrafiltration | 1-1.2 IGIU/mg protein |
| DEAE-Cellulose | 2.7 IGIU/mg protein |
| Gel Filtration | n.d. |

Once purified the glucose isomerase was immobilized according to the procedures in U.S. Patent 4,355,717 and also tested for thermal stability as follows:

40 ml of the partial purified glucose isomerase with total activity 474 IGIU (11.85 IGIU/ml) was adjusted to pH 7.2, then 6.61g d.b GDC (Granular Derivatized Cellulose [DEAE]) was added to the enzyme and mixed for 19 hours at room temperature, in 0.02% sodium azide. When the enzyme was 100% adsorbed, the immobilized enzyme activity was found to be 72 IGIU/g dry weight basis. The immobilized glucose isomerase (GDCI) was tested for thermo-stability at 98°C and 100°C using 0.51 g/ml substrate (glucose), 10mM $MgSO_4$, 10mM $NaHSO_3$ and 0.5mM $FeSO_4$, by monitoring the effluent with a polarimeter for fructose concentration. The results showed a halflife of 16.8 minutes at 100°C and 33.6 minutes at 98°C and Ea 100 Kcal/mole.

## Claims

16

1. An enzyme capable of the isomerization of glucose to fructose characterized by possessing a half-life of twenty minutes ($T_{20}$) at a temperature of at least 93°C.

2. The enzyme according to Claim 1 wherein said $T_{20}$ is from about 94°C to about 125°C.

3. The enzyme according to Claim 1 or 2 wherein said enzyme is immobilized onto an insoluble carrier.

4. The enzyme according to Claim 3 wherein said insoluble carrier is granular diethylaminoethyl cellulose (GDC).

5. The enzyme according to any of Claims 1 to 4 wherein the enzyme is isolatable from a member of the genus Bacillus

6. A process for isomerizing glucose to fructose which comprises contacting a glucose-containing feed liquor containing from 20 to about 64 weight percent glucose with a glucose isomerase at a temperature of from about 90°C to about 130°C at a pH from about 3.5 to about 8 and a contact time of from about 10 seconds to about 5 hours to convert at least about 53 to about 60 weight percent of the glucose present in said liquor to fructose with no substantial formation of psicose, characterized by employing a glucose isomerase possessing a half-life of twenty minutes ($T_{20}$) at a temperature of at least 93°C.

7. The process according to Claim 6 wherein said $T_{20}$ is from about 94°C to about 125°C.

8. The process according to Claim 6 or 7 wherein said glucose isomerase is chemically stabilized or said glucose isomerase is immobilized onto an insoluble carrier.

9. The process according to Claim 8 wherein said insoluble support is granular diethylaminoethyl cellulose (GDC).

10. The process according to any of Claims 1 to 9 wherein said glucose isomerase is isolatable from a member of the genus Bacillus.

11. A microorganism in biologically pure form, which produces an enzyme capable of the isomerization of glucose to fructose characterized by possessing a half-life of twenty minutes ($T_{20}$) at a temperature of at least 93°C.

12. The microorganism of Claim 11 wherein the microorganism is a member of the genus Bacillus.

FIG. 1
ARRHENIUS DIAGRAM FOR THE THERMAL INACTIVATION OF
GLUCOSE ISOMERASE

FIG.2

FIG.3

FIG.4a

FIG.4b

FIG.4c

FIG.4d

0    1/2    1 MILE        N↑

FIG.4e